# EUROPEAN PATENT APPLICATION

(11) **EP 4 467 176 A1**
(43) Date of publication of application: **27.11.2024**
(21) Application number: 23305846.0
(22) Date of filing: 26.05.2023
(51) Int. Cl.: A61M 5/19, A61M 5/20

(54) **DEVICE AND KIT FOR DELIVERING MEDICINE**

(71) Applicant: Eveon, 38330 Montbonnot-Saint-Martin (FR)
(72) Inventor: BOUILLET, Adrien, 38330 Montbonnot-Saint-Martin (FR); BACIOTTO, Tom, 38330 Montbonnot-Saint-Martin (FR)
(74) Representative: Icosa

(57) **Abstract**

The present invention relates to delivery device, kit and method for delivering drugs from two different containers.

## Description

### FIELD OF INVENTION

The present invention relates to a delivery device, kit and method. More precisely, it relates to a delivery and mixing device for medicine.

### BACKGROUND OF INVENTION

Nowadays, a considerable number of medical preparations are extemporaneous preparation. Extemporaneous preparations are classically known as preparations comprising at least one very (re)active, instable and/or very sensitive compound which therefore need to be prepared right before being administrated to the patient.

Extemporaneous preparation of emulsions and suspensions has increased over the past decade. Those type of formulati on enable the formation of a depot, which releases slowly over time to permit less frequent administration, and also decelerates drug degradation. They are designed to increase medication adherence and consistency, especially in patients who commonly forget to take their medicine. Depending on the formulation it is possible to switch from daily injection to monthly injections as example. This strategy is particularly interesting for, antipsychotics, hormonal, or antiviral treatment as example. This can also be of interest for vaccine preparation.

More specifically, the interest for the preparation of vaccines in oil emulsions increased over the past decade. This formulation is particularly advantageous for antigens which need an increased clinical response rate. This type of formulation is classically prepared by a user manipulating two syringes (syringe A and syringe B) end-to-end connected by an intermediary connector. A specific process needs to be put into practice by the user and can be summarized as follows:
- forcing a small amount of adjuvant from syringe B to syringe A by pressing the syringe B plunger,
- slowly pushing back the same volume of the mix from syringe A back to syringe B.

The two previous steps can be repeated until the mixed portion has become cloudy, for example milky white. Then, the user needs to increase the volume by small amounts and each time do the same mixing until final completion. Such manual preparation requires intense training and nerve wrecking attention in order to guarantee an equal peptide distribution in the emulsion while being highly repeatable and reproducible. The preparation quality shall not be influenced by the user nor by the change of material. Considering that the easiest preparation requires several minutes and over 20 back and forth movements, a technical solution is needed to save preparation time and improve the repeatability of the preparation.

Similar issues exist for the production of suspension type formulations (including nanosuspensions). This type of formulations is highly advantageous as nanoparticles enable higher drug loadings than other possible approaches. It is also highly advantageous to administer insoluble or poorly soluble drugs as suspensions rather than in their liquid formulation counterpart, as suspension usually leads to higher drug stability. Thus, suspension type formulations are highly advantageous for long-acting injectable medicines. Even though advance was made in the field of particle processing technologies to generate nanoparticle suspensions, it is highly difficult to obtain satisfying formulation with sufficient stability during the period between manufacture and use. For the Formulator, one of the main concerns is to permit easy re-suspensibility of settled particulate matter but also to decrease the sedimentation rate. If the solid particles, which gradually settle, cake too fast inside the syringe, this can cause difficulties or inefficient administration. To overcome this challenge, suspensions may require a reconstitution step just prior use. Thus, there may be chances of non-uniformity of dose at the time of administration if the reconstitution is not properly achieved by healthcare workers or the patient himself.

Besides, during the past decades, significant progress was made in the field of cell-based therapy. However few improvements were brought in the field of tools and protocol standardization to use for administration even if those steps are highly critical to improve cells survival rates.

For the most difficult cases, depending on the molecule to emulsify, it can be difficult to obtain a manual preparation stable through time with highly repeatable processes. Considering that the easiest preparation requires few minutes and over 20 back and forth movements, it is an objective of the present invention to propose a solution to healthcare workers (or patients) in order to automatize this preparation step. Due to physicochemical property such as molecule size, salts, polarity, it can be necessary to apply much higher speed and force than what is manually possible in order to obtain stable emulsions with high repeatable and reproducible processes.

Then after preparation, the system needs to be disconnected prior connecting a syringe for performing manual injection. This traditional user scenario is prone to sharp injuries and sterility breakage. It is required for the user to provide a device with increased safety and reduced number of steps for performing injection.

The objective of this invention is to overcome those two challenges, in a single device enabling mixing and injection in an easy and safe way available to any health worker and most patients. This way, the invention aims at overcoming those challenge in the most portable way by providing a compact handheld device.

### SUMMARY

This invention thus relates to a delivery device for delivering a composition resulting from a mixture of two products contained respectively in two containers, each container comprising an outlet and a mobile extremity opposite the outlet, the delivery device (10) further extending along an elongation axis, said device comprising:
- first and second housings each configured to secure one container along the elongation axis,
- first and second activation elements, each configured to move back and forth along an activation axis, the first activation element being configured to cooperate with the mobile extremity of the container secured by the first housing and the second activation element being configured to cooperate with the mobile extremity of the container secured by the second housing,
- a fluidic system comprising:
   ∘ administration means,
   ∘ first and second ports configured to be connected respectively to the outlets of the containers,
   ∘ a first fluidic channel configured to put the first and second ports in fluidic communication,
   ∘ a second fluidic channel configured to put the administration means in fluidic communication with the first port,
   ∘ a switch configured to adopt a first position opening the first fluidic channel and closing the second fluidic channel, and a second position opening the second fluidic channel and closing the first fluidic channel,
- a driving system configured to generate a rotational movement,
- transmission means connecting the driving system to each of the first and second activation elements,

**wherein** each of the activation axis extends parallel to the elongation axis, each activation axis extending in a distinct plane,
**wherein** the transmission means is configured to transform the rotational movement of the driving system into the back-and-forth movement of the activation elements, the back-and-forth movement of the activation elements being asynchronous.

This way, the invention enables to reach the here-above mentioned objective. Especially, it enables the standardization of complex fluid preparation prior use. In the present application, complex fluid means, liquid-solid preparation (suspensions), or liquid-liquid preparation (emulsion) or viscous fluid such as gel or hydrogel. The term standardization means that this invention allows a perfect control of product preparation regarding at least time, product handling and speed in order to obtain highly repeatable results compare to manual preparation made by healthcare workers, patients, or trained laboratory workers as example. The present invention further enables to adjust the dose to be delivered as it is, indeed, often necessary to prepare a dose but adjusting the volume delivered according age, body weight or condition of the patient. As example it is necessary to have the possibility to deliver only 1, 1, or 2mL of drug even though 4ml have been prepared.

The delivery device according to the invention may include one or more of the following characteristics, taken separately from one another or in combination with one another:
- each of the first and second activation elements extend between an actuating end configured to be in contact with the mobile extremity of the container, and a driven end, and wherein the transmission means comprises a cam disc configured to be driven in rotation about the elongation axis by the driving system, the came disk comprising a guiding surface around the elongation axis, the guiding surface being inclined with respect to the elongation axis and receiving the driven end of the first and second activation elements,
- the driving system comprises a driving member configured to impart the rotational movement continuously in one direction about a driving axis parallel to the elongation axis,
- each of the first and second activation elements extend between an actuating end configured to be in contact with the mobile extremity of the container, and a driven end, and wherein the driving system comprises a driving member configured to impart the rotational movement alternatively in opposite directions about a driving axis perpendicular to the elongation axis, the transmission means comprising connecting members each extending along one of the activation axes and connecting the driven end of one of the activation elements to the driving member,
- the transmission means is configured so that the first activation element is in a bottom end position when the second activation element is in a top end position, and the first activation element is in a top end position when the second activation element is in a bottom end position,
- the delivery device comprises a casing configured to accommodate at least the first and second housings, the first and second activation elements, the fluidic system and the transmission means, and wherein the fluidic system is removable from the casing,
- the casing comprises a body extending along the elongation axis from an access opening, and a head cap configured to reversibly close the access opening, the body accommodating the first and second housings, the first and second activation elements and the transmission means, the head cap comprising an attaching arrangement configured to removably attach the fluidic system to said head cap,
- the casing is further configured to accommodate the driving system and an autonomous power source for supplying the driving system with energy,
- the delivery device comprises a docking station configured to accommodate the driving system and a power source for supplying the driving system with energy, the docking station presenting a support surface configured to removably receive the casing, the support surface being arranged to connect the transmission means with the driving system when the casing is placed on the support surface,
- the administration means is chosen among an injection element such as a needle, and a nebulizer,
- the driving means comprises an electric motor comprising a rotating shaft connected to the transmission means,
- the driving system comprises a control unit including an encoder configured to emit a signal representative of a stroke of the motor, said control unit being configured to associate a determined administration dose with the stroke of the motor,
- the driving system further comprise a stopping element configured to restrain the back-and-forth movements of the first and second activation elements,
- the first and second housings comprise a container connection distance regulation system configured to secure the containers at a given distance respectively from the first and second ports in order to regulate positions of the mobile extremities of said containers with regards to the first and second housings and the first and second activation elements, so as to regulate a length of the back-and-forth movements of said first and second activation elements.

A further object of the present invention is a delivery kit comprising a delivery device according to any one of the preceding claims, and at least two containers configured to be received respectively in the first and second housing, each container comprising an outlet and a mobile extremity opposite the outlet, the mobile extremity being configured to cooperate with one of the first and second activation elements.

A last object of the present invention is a delivery method for delivering a composition resulting from a mixture of two products contained respectively in two containers, each container comprising an outlet and a mobile extremity opposite the outlet, the delivery method implementing the delivery device according to any one of the here-above listed features and comprising steps of:
- connecting the outlets of the containers respectively to first and second ports of the fluidic section,
- securing the containers to the first and second housings along the elongation axis,
- actuating the driving means to impart back-and-forth movements to the first and second activation elements through the transmission means, the back-and-forth movements of the first and second activation elements being asynchronous, the first activation element being in a bottom end position while the second activation element is spaced apart from the bottom end position.

### BRIEF DESCRIPTION OF THE DRAWINGS

The invention will be better understood, and other aims, details, characteristics and advantages thereof will emerge more clearly on reading the detailed explanatory description which follows, of embodiments of the invention given by way of illustration. purely illustrative and non-limiting examples, with reference to the accompanying drawings, in which:
- **figure 1** is a partially exploded view representing the different elements a user can disassemble in a normal use of a device according to the present invention,
- **figure 2** is a more exploded view of a device according to the same embodiment as figure 1,
- **figures 3A, 3B and 3B** are three sectional schematical views from a driving system and a transmission means 30 according to a first embodiment of the present invention,
- **figure 4** **is** a schematical view of transmission means according to a second embodiment of the present invention,
- **figures 5A and 5B** presents two schematic sectional views from the fluidic system according to a first embodiment of the present invention,
- figure 6 is a perspective view of the inside of the embodiment of figure 1,
- figure 7 is a perspective view of another embodiment of the present invention.

### DETAILED DESCRIPTION

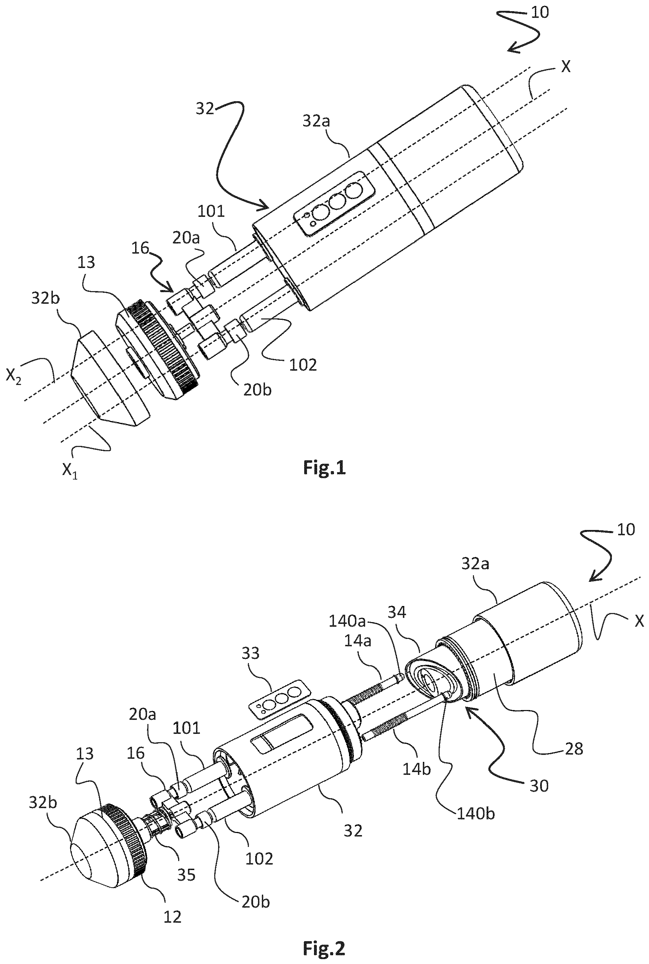

As can be seen on figures 1 and 2, the present invention is about a delivery device 10 for delivering a composition resulting from a mixture of two products contained respectively in two containers 101, 102. The delivery device 10 can also be a mixing device. The delivery device 10 extends along an elongation axis X.

According to the present invention, the delivery device 10 comprises:
- first and second housings 12a, 12b each configured to secure one container 101, 102, along the elongation axis X,
- first and second activation elements 14a, 14b, each configured to move back and forth along an activation axis X₁, X₂ between a top end position and a bottom end position,
- a fluidic system 16 comprising:
   ∘ administration means 18,
   ∘ first and second ports 20a, 20b configured to be connected respectively to the outlets of the containers 101, 102,
   ∘ a first fluidic channel 22,
   ∘ a second fluidic channel 24, distinct from the first fluidic channel 22,
   ∘ a switch 26 configured to adopt a first position opening the first fluidic channel 22 and closing the second fluidic channel 24, and a second position opening the second fluidic channel 24 and closing the first fluidic channel 22,
- a driving system 28 configured to generate a rotational movement,
- transmission means 30 connecting the driving system 28 to each of the first and second activation elements 14a, 14b.

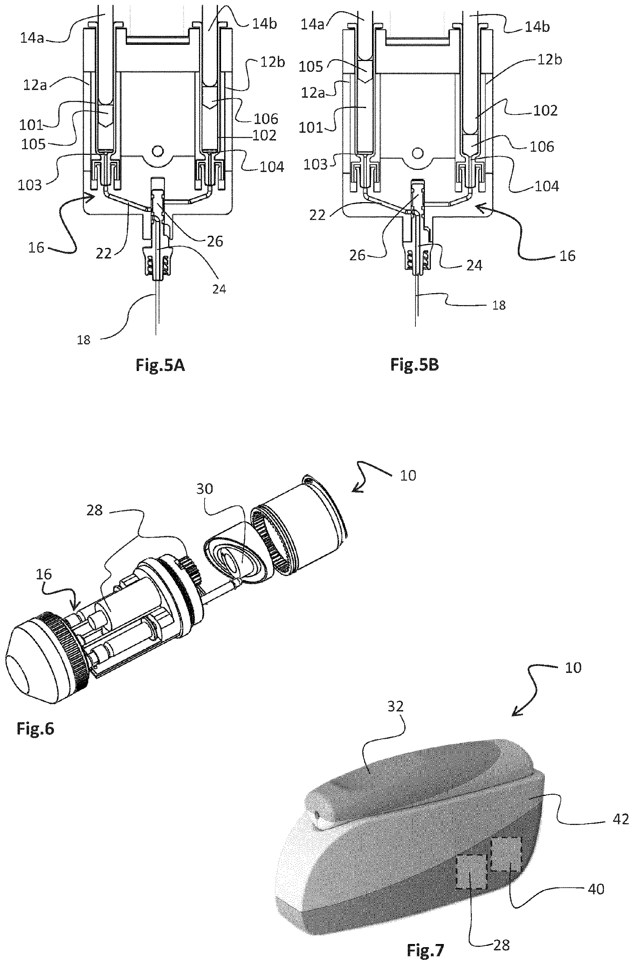

Each container 101 102 preferably presents an elongated shape extending along a central axis. Each container 100a, 100b presents an outlet 103, 104, preferably an axial outlet and a mobile extremity 105, 106 opposing the outlet 103, 104. Each of the two containers 101, 102 thus comprises a hollow body and the mobile extremity 105, 106 is mobile in translation along the central axis inside the hollow bodies. Each mobile extremity 105, 106 thus defines, within each container 101, 102, a variable volume chamber. Each of the containers 101, 102 is further configured to cooperate with an associated activation element 14a, 14b to translate the mobile extremity inside the body and thus vary the volume of the chamber. More precisely, the first activation element 14a is configured to cooperate with the mobile extremity of the container 101 secured by the first housing 12a and the second activation element 14b is configured to cooperate with the mobile extremity of the container 102 secured by the second housing 12b. Each one of the mobile extremities 105, 106 of the containers 101, 102 may comprise an actuating end 141, 142 (preferably being an elongated element) configured to be in contact with the mobile extremity 105, 106 of the containers 101, 102. A typical elongated element (actuating end 141, 142) is for example, a piston. The elongated containers 101, 102 might be a syringe or a cartridge (see figures 5A and 5B).

The administration means 18 is preferably an injection element, such as, for example, a needle or. In an alternative embodiment, the administration means could be a nebulizer or an atomization nozzle. Such a device with a nebulizer or a nozzle can be particularly interesting for extemporaneous preparation of highly viscous gel having specific rheologic properties (shear thinning), thermogels, etc.

The housings 12a, 12b extend along the activation axis X₁, X₂ in order to align each container 101, 102 with the elongation axis X. The housings 12a, 12b preferably present a concave shape with regards to the elongation axis X, configured to receive the containers 101, 102. In some embodiments, the first and second housings 12a, 12b are configured to secure the containers 101, 102 at a precise distance from the first and second ports 20a, 20b of the fluidic system 16. More precisely, in those embodiments, each housing 12a, 12b comprises a "container connection distance regulation system" or distance regulator 13 configured to secure each container 101, 102 at a given distance from the its corresponding port 20a, 20b. The distance regulator 13 can for example be, as can be seen on figure 1 and 2, a notched wheel which can be screwed and unscrewed along the elongation axis X thus enabling to adjust the position of the housing 12a, 12b along said elongation axis X. The distance regulator 13 can also be adjusted along the elongation axis X by means of a telescopic or translating displacement from at least two positions: a fully retracted position which enables a full stroke and at least on intermediate position corresponding to one of the different possible strokes. More precisely, by unscrewing or adjusting the distance regulator 13, prior administration, the user adjusts the stroke of the activation elements 14a, 14b (piston stroke) and this allows the user to adjust the dose to the patient.

This enables the regulation of the positions of the mobile extremities 103, 104 of the containers 101, 102 with regards to the first and second housings 12a, 12b and the first and second activation elements 14a, 14b, so as to regulate a length of the back-and-forth movements of said first and second activation elements 14a, 14b Adjusting the insertion height of the containers 101, 102 inside the housings 12a, 12b enables to adjust the length of the translation (back and forth movements) and renders possible to reduce (or increase) the volume of fluid to be administrated to the patient.

It is preferable for a better mixing quality to allow a complete mixing between the volumes of containers 101, 102. This implies an equivalent stroke between the two containers 101, 102. Nevertheless, sometimes two reactants to be mixed are contained in two heterogeneous containers 101, 102. In this case the mixture will be done by partial transfer from one container to another 101, 102.

The fluidic system 16 comprises channels with a diameter size ranging from 0.2 mm to 2mm. Those channels may be made of plastic, glass or other biocompatible material suitable for medical applications. Diameter size of the fluidic system 16 as well as material used for its manufacturing can be designed on purpose depending on the product to be mixed (e.g biochemical properties, rheological properties...). The fluidic system 16 may be an independent element of the device 10 which can be removably connected to the housings 12a, 12b, as can be seen on figure 1. In an alternative embodiment, nevertheless, the fluidic system 16 is part of a platform also comprising, for example, the housings 12a, 12b (see figures 5A and 5B). In those embodiments, the fluidic system 16 might be directly carved inside said platform.

In order to confer some mechanical protection, the device 10 further preferably comprises a casing 32 configured to accommodate at least the first and second housings 12a, 12b, the first and second activation elements 14a, 14b, the fluidic system 16 and the transmission means 28. A casing 32 can also ease the handling of the device 10 by offering an ergonomically adapted shape. This way, the device 10 can be operated either by a healthcare worker or directly by a patient. The casing 32 can also present an open window, or can be made of transparent material in order to enable visual control of proper mixing and functioning of the device 10. The head cap 32b also prevents any user to experience any uncontrolled administration: the device 10 can only be used when the head cap 32b is removed. Sensors can be implemented in order to lock the device 10 at an interface between the distance regulator 13 and the casing 32, and/or between the head cap 32b and distance regulator 13 during the mixing and thus preventing any possible inadvertent opening or activation of the administration means 18. Administration can also be prevented by programming and being dependent of prior mixing step.

In some embodiments, the casing may comprise a button which renders possible to mechanically adjust the volume of fluid to be administered either by blocking the path of the transmission means 30, or directly adjusting the containers 101, 102 insertion eight.

If the fluidic system 16 is an independent element, the fluidic system 16 is preferably removable from the casing 32. This enables to easily change the fluidic system 16 without the need of discarding all the other functional elements of the device 10. This further leads the device 10 to comprise a reusable and a disposable part, and to discarded the disposable part without the user gaining access to the driving system 28, the transmissions means 30 and the activation elements 14a, 14b for safety measures.

Preferably, the casing 32 comprises (see figures 1 and 2):
- a body 32a accommodating the first and second housings 12a, 12b, the first and second activation elements 14a, 14b and the transmission means 30, the body 32a extending along the elongation axis X from an access opening enabling to insert the containers 101, 102 inside the housings 12a, 12b,
- the distance regulator 13, and
- a removable head cap 32b configured to reversibly close the access opening of the body 32a.

In some embodiments, the casing 32 may further present a user interface 33 to enable an easy use of the device 10 and the easy control and/or monitoring of the activation elements 14a, 14b.

If the fluidic system 16 is an independent element, the removable head 32b also preferably comprises an attaching arrangement configured to removably attach the fluidic system 16 to it. It may also comprise a protecting device 35 for protecting the administration means 18

As already mentioned, the fluidic system 16 comprises two distinct fluidic channels 22, 24:
- a first fluidic channel 22 configured to put the first and second ports 20a, 20b in fluidic communication with each other, thus enabling the variable volume chambers of the container 101, 102 to be put in fluidic communication,
- a second fluidic channel 24, distinct from the first fluidic channel 22, configured to put the administration means 18 in fluidic communication with the first port 20a.

The first fluidic channel 22 thus enables the mixing of any reactants contained inside the first and second containers 101, 102, while the second fluidic channel 24 enables the delivery of the fluid present inside the first container 101 through the administration means 18. The position of the switch 26 determines which fluidic channel 22, 24 is open and thus enables:
- either to mix the different reactants contained in the containers 101, 102 in enabling the fluidic components of said reactants to be displaced from the first container 101 to the second container 102 and *vice versa,* or
- to deliver any fluid (preferably resulting from a mixing between the reactants of the first and second containers 101, 102) contained in the first container 101.

Said switch 26 might be a valve or a distributor comprising a mechanical fluid stopping element and/or a mechanical fluidic junction element. The fluidic switching can be done by:
- injection pressure, and/or
- external actuation of the device 10 by means of a prior action from the user like pressing a button, pressing a command, etc., and/or
- driven by a second engine and be fully automated.

Preferably, the first fluidic channel 22 passes around the switch 26 and the second fluidic channel 24 is integrated to the switch 26, as can be seen on figures 5A and 5B.

In case the device 10 comprises a casing 32, the first fluidic channel 22 is integrated in the casing 32 and the second fluidic channel 24 is integrated into the casing 32 and the switch 26.

Classically, any fluid contained in the first and second containers 101, 102 is put in motion through one or the other fluidic channels 22, 24 by the motion of the mobile extremities 105, 106 of each container 101, 102 within the hollow bodies of the containers 101, 102.

This mobility is achieved by the combined action of the driving system 28, the transmission means 30 and the activation means 14a, 14b. The driving system 28 drives the transmission means 30 which drives the actuation means 14a, 14b which drive the mobile extremities 105, 106 of the containers 101, 102. More precisely, each of the first and second activation elements 14a, 14b extend between a corresponding actuating end 141, 142 configured to cooperate with the mobile extremity 105, 106 of the containers 101, 102 (see above), and a corresponding driven end 140a, 140b configured to cooperate with the transmission means 30. Thus, the transmission means 30 are configured to transform the rotational movement of the driving system 28 into the back-and-forth movement of the activation elements 14a, 14b.

In order to enable the proper mixing of the reactants contained inside the containers 101, 102, the back-and-forth movement of the activation elements 14a, 14b is asynchronous, meaning that the first activation element 14a is in the bottom end position while the second activation element 14b is spaced apart from the bottom end position. In other words, the transmission means 28 is configured to lead first activation element 14a to be in the bottom end position when the second activation element 14b is in the top end position, and the first activation element 14a to be in the top end position when the second activation element 14b is in the bottom end position. And eventually, the displacement of the first and second activation elements 14a, 14b along their activation axis X₁, X₂ induces a corresponding displacement of the mobile extremities 105, 106 of the containers 101, 102.

In some embodiments, the driving system 28 comprises an electric motor comprising a rotating shaft connected to the transmission means 30. This way, the mixing of the reactants of the two containers 101, 102 can be done in an automatic way. This automatic way can achieve quicker and faster mixing than a human mixing. The driving system 28 may also comprise a control unit including an encoder. More precisely, the use of a motor encoder permits to adjust the dose according to the position of the transmission means 30. A position is correlated to the length of a translation (back and forth movement). It is then possible to use a device software to regulate volume. Such a control unit enables a great precision and increases safety. Said control unit is also configured provide feedback control over the motor according the measure stroke. Thus, it is possible to detect potential blockage during mixing and reverse the motor's rotation in order to have back and forth mixing thanks to partial strokes of activations elements 14a and 14b. A further role of the encoder is the potential detection of a powder jam inside one of the two containers 101, 102 during the mixing of the reactants, and thus reduce the stroke of the activation elements 14a and 14b in order to avoid any kind of break or damage and induce and favorize a smooth and homogeneous mixing. The encoder thus has two different roles:
- either detect a powder jam and enable a reduction of the back-and forth movement of the activation elements 14a, 14b,
- either perform an incomplete run for the activation elements 14a, 14b (as position on disk = position in translation = administered dose).

Importantly, each of the activation axis X₁, X₂ extends parallel to the elongation axis X, each activation axis X₁, X₂ extending in a distinct plane. This implicates that the housings 12a, 12b preferably extend side by side along the elongation axis X and thus, that the containers 101, 102 are secured side by side to the delivery device 10. This enables the asynchronous back and forth (translation) movements of the activation elements 14a, 14b.

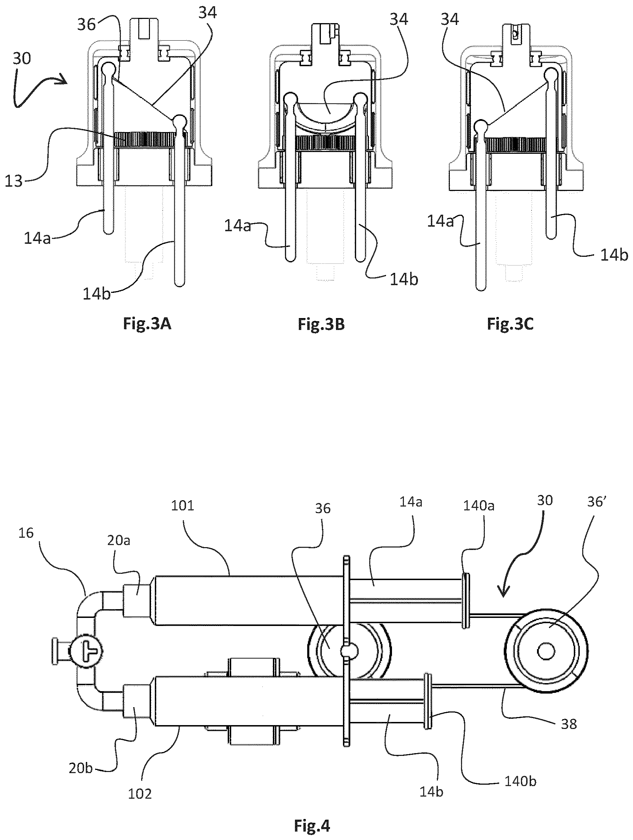

In a first embodiment depicted on figures 3A, 3B and 3C, the transmission means 30 comprises a cam disc 34 configured to be driven in rotation around the elongation axis X by the driving system 28. The came disk 34 comprises a guiding surface around the elongation axis X (for example a groove). This guiding surface is inclined with respect to the elongation axis X and receives the driven ends 140a, 140b of the first and second activation elements14a, 14b. The cam-disk 34 is constantly inclined with regards to the elongation axis X and thus, when the cam-disk 34 rotates around the elongation axis X, one specific point on the circumference of the cam-disk 34 will go "up and down" around the elongation axis X. This way, when the cam-disk 34 rotates, the activation elements 14a, 14b are driven in a back-and-forth movement along with the rotation of the cam-disk 34. The guiding surface may be a groove configured to cooperate with the driven ends 140a, 140b of the activation elements14a, 14b. The maintaining of the driven ends 140a, 140b can be obtained by returning spring. The guiding surface may further comprise a specific profile which avoids the driven ends 140a, 140b to disconnect from the guiding surface (in case of a groove, to fall out or fall off the groove).

The inclination of the guiding surface can be decided with regards to the desired stroke for the pistons. A stopping element enabling blockage within the cam disc 34, configured to restrain the stroke activation elements 14a, 14b' during administration can be implemented in order to adjust the dose to be delivered. The stopping element can be actuated by an external button from the user, or electronically actuated thanks to the user interface 33.

This cam-disc transmission system benefits from continuous motor rotation for mixing with no pauses and enables to achieve a high-speed mixing.

In this embodiment, the driving system 28 may comprise a driving member 36 configured to impart the rotational movement continuously in one direction about a driving axis parallel to the activation axes X₁, X₂ parallel to the elongation axis X.

In an alternative embodiment (see figure 4), the driving system 28 comprises a driving member 36' (for example a pulley system comprising a strap) configured to impart the rotational movement alternatively in opposite directions around a driving axis Y perpendicular to the elongation axis X. In this embodiment, the transmission means 30 comprising connecting members 38 each extending along one of the activation axes X₁, X₂ and connecting each driven end 140a, 140b of the activation elements 14a, 14b to the driving member 36'. The connecting members 38 could for example be one strap or two distinct straps, rods or connecting links.

In the embodiments in which the device comprises a casing 32, the casing 32 may further be configured to accommodate the driving system 28. It also may comprise an autonomous power source 40 for supplying the driving system 28 with energy.

In some embodiments, the device 10 according to the present invention may further comprise a docking station 42 configured to accommodate the driving system 28 and the power source 40 for supplying said driving system 28 with energy (see figure 7). The docking station 42 presents a support surface configured to removably receive the casing 32 as described above. The support surface can comprise a groove or a print adapted to secure the device 10 and avoid any undesired lateral movement during the mixing of the reactants from the containers 101, 102. In this embodiment, the support surface of the docking station 42 is arranged to connect the transmission means 30 with the driving system 28 when the casing 32 is placed on it (see figure 7). This docking station 42 enables the device 10 to become a portable handheld device while outsourcing motorization. This enables to have a very strong motorization, able to mix highly viscous or difficult to mix drugs which cannot fit inside a handled device. Additionally, the docking station 42 increases the stability during the mixing.

The delivery device 10 according to the present invention is to be used in kit. This kit comprises a mixing and delivery device 10 according to the current invention, and at least two containers 101, 102 configured to be received respectively in the first and second housing 12a, 12b.

This kit enables to implement a delivery method for delivering a composition resulting from a mixture of two products contained respectively in two containers 101, 102 according to the present invention, the delivery method implementing the delivery device according to the present invention and comprising steps of:
- connecting the outlets 103, 104 of the containers 101, 102 respectively to first and second ports 20a, 20b of the fluidic system 16,
- securing the containers 101, 102 to the first and second housings 12a, 12b along the elongation axis X,
- actuating the driving means 28 to impart back-and-forth movements to the first and second activation elements 14a, 14b through the transmission means 30, the back-and-forth movements of the first and second activation elements 14a, 14b being asynchronous, the first activation element 14a being in the bottom end position while the second activation element 14b is spaced apart from the bottom end position.

In conclusion, the device 10 according to the present invention, the associated kit and method present actuation means compatible with all containers having displaceable plungers and is compatible with viscosities above 100Cp. Further the present invention enables to have a standardized preparation system in which any positions mixing is ensured with activations means enabling total emptying of containers. The device 10 according to the present invention further enables high speed mixing and possible mixing with controlled speed variations in order to provided homogeneous product prior administration.

## Claims

1. Delivery device (10) for delivering a composition resulting from a mixture of two products contained respectively in two containers (101, 102), each container (101, 102) comprising an outlet (103, 104) and a mobile extremity (104, 105) opposite the outlet (103, 104), the delivery device (10) further extending along an elongation axis (X), said device (10) comprising:
- first and second housings (12a, 12b) each configured to secure one container (101, 102) along the elongation axis (X),
- first and second activation elements (14a, 14b), each configured to move back and forth along an activation axis (X₁, X₂), the first activation element (14a) being configured to cooperate with the mobile extremity (105) of the container (101) secured by the first housing (12a) and the second activation element (14b) being configured to cooperate with the mobile extremity (106) of the container (102) secured by the second housing (12b),
- a fluidic system (16) comprising:
∘ administration means (18),
∘ first and second ports (20a, 20b) configured to be connected respectively to the outlets (103, 104) of the containers (101, 102),
∘ a first fluidic channel (22) configured to put the first and second ports (20a, 20b) in fluidic communication,
∘ a second fluidic channel (24) configured to put the administration means (18) in fluidic communication with the first port (20a),
∘ a switch (26) configured to adopt a first position opening the first fluidic channel (22) and closing the second fluidic channel (24), and a second position opening the second fluidic channel (24) and closing the first fluidic channel (22),
- a driving system (28) configured to generate a rotational movement,
- transmission means (30) connecting the driving system (28) to each of the first and second activation elements (14a, 14b),
**wherein** each of the activation axis (X₁, X₂) extends parallel to the elongation axis (X), each activation axis (X₁, X₂) extending in a distinct plane,
**wherein** the transmission means (30) is configured to transform the rotational movement of the driving system (28) into the back-and-forth movement of the activation elements (14a, 14b), the back-and-forth movement of the activation elements (14a, 14b) being asynchronous.

2. Delivery device (10) according to claim 1, wherein each of the first and second activation elements (14a, 14b) extend between an actuating end (141, 142) configured to be in contact with the mobile extremity (105, 106) of the container (101, 102), and a driven end (140a, 140b), and wherein the transmission means (14a, 14b) comprises a cam disc (34) configured to be driven in rotation about the elongation axis (X) by the driving system (28), the came disk (34) comprising a guiding surface around the elongation axis (X), the guiding surface being inclined with respect to the elongation axis (X) and receiving the driven end (140a, 140b) of the first and second activation elements (14a, 14b).

3. Delivery device (10) according to claim 2, wherein the driving system (28) comprises a driving member (36) configured to impart the rotational movement continuously in one direction about a driving axis parallel to the elongation axis (X).

4. Delivery device (10) according to claim 1, wherein each of the first and second activation elements (14a, 14b) extend between an actuating end (141, 142) configured to be in contact with the mobile extremity (105, 106) of the container (101, 102), and a driven end (140a, 140b), and wherein the driving system (28) comprises a driving member (36) configured to impart the rotational movement alternatively in opposite directions about a driving axis perpendicular to the elongation axis (X), the transmission means (30) comprising connecting member each extending along one of the activation axes and connecting the driven end (140a, 140b) of one of the activation elements (14a, 14b) to the driving member (36).

5. Delivery device (10) according to any of claims 1 to 4, wherein the transmission means (30) is configured so that the first activation element (14a) is in a bottom end position when the second activation element (14b) is in a top end position, and the first activation element (14a) is in a top end position when the second activation element (14b) is in a bottom end position.

6. Delivery device (10) according to any one of the preceding claims, further comprising a casing (32) configured to accommodate at least the first and second housings (12a, 12b), the first and second activation elements (14a, 14b), the fluidic system (16) and the transmission means (30), and wherein the fluidic system (16) is removable from the casing (32).

7. Delivery device (10) according to claim 6, wherein the casing (32) comprises a body (32a) extending along the elongation axis (X) from an access opening, and a head cap (32b) configured to reversibly close the access opening, the body (32a) accommodating the first and second housings (12a, 12b), the first and second activation elements (14a, 14b) and the transmission means (30), the head cap (32b) comprising an attaching arrangement configured to removably attach the fluidic system (16) to said head.

8. Delivery device (10) according to any one of claims 6 and 7, wherein the casing (32) is further configured to accommodate the driving system (28) and an autonomous power source (40) for supplying the driving system (28) with energy.

9. Delivery device (10) according to any of claims 6 and 7, further comprising a docking station (42) configured to accommodate the driving system (28) and a power source (40) for supplying the driving system (28) with energy, the docking station (42) presenting a support surface configured to removably receive the casing (32), the support surface being arranged to connect the transmission means (30) with the driving system (28) when the casing (32) is placed on the support surface.

10. Delivery device (10) according to any one of the preceding claims, wherein the administration means (18) is chosen among an injection element, such as a needle, and a nebulizer.

11. Delivery device (10) according to any one of the preceding claims, wherein the driving means (30) comprises an electric motor comprising a rotating shaft connected to the transmission means (30).

12. Delivery device (10) according to the preceding claim, wherein the driving system (28) comprises a control unit including an encoder configured to emit a signal representative of a stroke of the motor, said control unit being configured to associate a determined administration dose with the stroke of the motor.

13. Delivery kit comprising a delivery device (10) according to any one of the preceding claims, and at least two containers (101, 102) configured to be received respectively in the first and second housing (12a, 12b), each container (101, 102) comprising an outlet and a mobile extremity (105, 106) opposite the outlet, the mobile extremity (105, 106) being configured to cooperate with one of the first and second activation elements (14a, 14b).

14. Delivery method (10) for delivering a composition resulting from a mixture of two products contained respectively in two containers (101, 102), each container (101, 102) comprising an outlet and a mobile extremity (105, 106) opposite the outlet, the delivery method implementing the delivery device according to any of claims 1 to 14 and comprising steps of:
- connecting the outlets of the containers (101, 102) respectively to the first and second ports of the fluidic system (16),
- securing the containers (101, 102) to the first and second housings (12a, 12b) along the elongation axis (X),
- actuating the driving means (28) to impart back-and-forth movements to the first and second activation elements (14a, 14b) through the transmission means (30), the back-and-forth movements of the first and second activation elements (14a, 14b) being asynchronous, the first activation element (14a) being in a bottom end position while the second activation element (14b) is spaced apart from the bottom end position.
